(19) Europäisches Patentamt
European Patent Office
Office européen des brevets

(11) **EP 4 029 437 A1**

(12) **EUROPEAN PATENT APPLICATION**

(43) Date of publication:
**20.07.2022 Bulletin 2022/29**

(21) Application number: **21151401.3**

(22) Date of filing: **13.01.2021**

(51) International Patent Classification (IPC):
*A61B 5/024* (2006.01)     *A61B 5/021* (2006.01)
*A61B 5/08* (2006.01)      *A61B 5/0205* (2006.01)
*A61B 5/00* (2006.01)

(52) Cooperative Patent Classification (CPC):
**A61B 5/02416; A61B 5/0205; A61B 5/02108;
A61B 5/0816; A61B 5/721; A61B 5/7239;
A61B 5/7246; A61B 5/7267; A61B 5/7278**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **DE GROOT, Koen Theo Johan
5656 AE Eindhoven (NL)**
• **HAAKMA, Reinder
5656 AE Eindhoven (NL)**
• **GELISSEN, Jozef Hubertus
5656 AE Eindhoven (NL)**

(74) Representative: **Philips Intellectual Property &
Standards
High Tech Campus 52
5656 AG Eindhoven (NL)**

(54) **BLOOD PRESSURE ESTIMATION**

(57)     According to an aspect, there is provided a computer-implemented method of estimating blood pressure of a subject. The method comprises (i) processing (101) a first blood volume signal to identify a plurality of blood volume signal segments each corresponding to a respective cardiac cycle of the subject, wherein the first blood volume signal comprises measurements of blood volume or changes in blood volume in a body part of the subject in a first time period; (ii) processing (103) a first respiration signal to identify a plurality of respiration cycles of the subject in the first time period, wherein the first respiration signal represents respiratory activity of the subject during the first time period, and wherein each respiration cycle comprises a plurality of respiration cycle phases; (iii) selecting (105) a subset of the plurality of blood volume signal segments, wherein the blood volume signal segments selected for the subset are blood volume signal segments corresponding in time to a same respiration cycle phase in the plurality of respiration cycles; and (iv) estimating (107) blood pressure of the subject based on morphology features of the blood volume signal segments in the subset.

Process a first blood volume signal to identify a plurality of blood volume signal segments, each corresponding to a respective cardiac cycle of the subject — 101

Process a first respiration signal to identify a plurality of respiration cycles of the subject in the first time period — 103

Select a subset of the blood volume signal segments corresponding in time to a same respiration cycle phase in the plurality of respiration cycles — 105

Estimate blood pressure of the subject based on morphology features of the blood volume signal segments in the subset — 107

Fig. 7

Processed by Luminess, 75001 PARIS (FR)

**Description**

FIELD OF THE INVENTION

This disclosure relates to estimating blood pressure of a subject, and in particular to a computer-implemented method, a computer program product and an apparatus for estimating blood pressure non-invasively from blood volume signals.

BACKGROUND OF THE INVENTION

It is anticipated that the development of health-related unobtrusive sensing systems will enable a shift from conventional hospital treatment to monitoring of a patient or subject using unobtrusive vital signs sensor technologies. It is expected that such vital signs monitor systems will provide better information about the subject's general health condition, reduce treatment costs through disease prevention, enhance quality of life, and improve availability of physiological data to enable physicians to better diagnose the subject's general health condition. Vital signs monitoring typically includes monitoring one or more of the following physical parameters: heart rate (pulse rate), blood pressure, respiratory rate (breathing rate) and core body temperature.

In the US about 30% of the adult population has a high blood pressure (hypertension). Only around half of this population have their condition under control. Hypertension is a common health problem which has no obvious symptoms, but which may ultimately cause death. Blood pressure generally rises with age, and the risk of becoming hypertensive in later life is considerable. Persistent hypertension is one of the key risk factors for strokes, heart failure and increased mortality. The condition of subjects with hypertension can be improved through lifestyle changes, healthy dietary choices and medication. Particularly for high-risk patients, continuous (e.g. 24-hour) blood pressure monitoring can be very useful. However, it is desirable for this monitoring to be performed by means of systems that do not impede ordinary daily life activities.

Blood pressure is usually measured as two readings: systolic pressure and diastolic pressure, and presented as 'systolic/diastolic' or 'systolic over diastolic'. Systolic pressure occurs in the arteries during the maximal contraction of the left ventricle of the heart. Diastolic pressure refers to the pressure in arteries when the heart muscle is resting between beats and refilling with blood. Normal blood pressure is considered to be less than 120/80 mmHg. A person is generally considered to be hypertensive when their blood pressure is above 140/90 mmHg.

There are two main classes of methods for monitoring blood pressure. The first is invasive direct blood pressure monitoring. The second is non-invasive indirect blood pressure monitoring.

Of the invasive direct blood pressure (BP) monitoring, the gold standard to measure BP is by catheterisation. A strain gauge in fluid is placed in direct contact with blood at any artery site. This method is only used when accurate continuous blood pressure monitoring is required in dynamic (e.g. clinical) circumstances. It is most commonly used in intensive care medicine and anaesthesia to monitor blood pressure in real time.

There are several non-invasive indirect blood pressure monitoring techniques that can be used, including those based on oscillometry, pulse wave velocity (PWV), pulse wave morphology, auscultation, tonometry and ultrasound measurements.

Pulse wave morphology-based techniques estimate BP from a set of features that describe the morphology of the PPG wave, since several morphological properties of the PPG waveform are considered to correlate with blood pressure.

Some existing PPG morphology feature extraction methods for estimating BP are set out below.

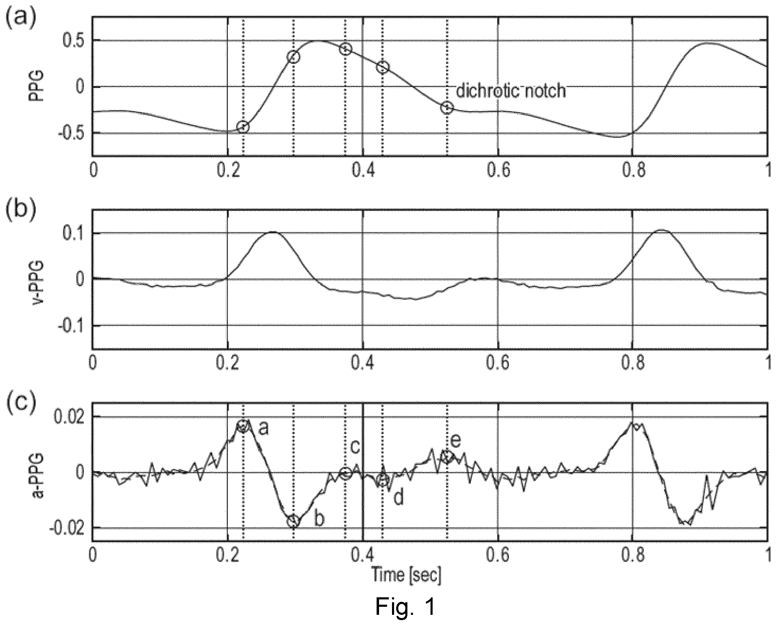

In a first set of methods, landmark features of the PPG signal are used. In the paper "On the analysis of fingertip photoplethysmogram signals" by Elgendi, Current Cardiology Reviews, 2012, a method is explained to extract 2nd derivative features from a PPG beat interval. Firstly, a robust 2nd derivate (derivative) representation of the PPG beat interval is derived. Next, significant landmarks are detected in the PPG signal using the 2nd derivative signal, as shown in Fig. 1.

Thus, Fig 1(a) shows an exemplary PPG signal covering a 1-second period with the dicrotic notch indicated. Fig. 1(b) shows a first derivative of the PGG signal of Fig. 1(a) with respect to time (denoted v-PPG), and Fig. 1(c) shows a second derivative of the PGG signal of Fig. 1(a) with respect to time (denoted a-PPG). The 'acceleration' waveform (a-PPG) can be considered to consist of five 'fiducial points' or 'reference points', as shown in Fig 1(c). The *a* point marks the start of the direct pulse wave and the *b* point marks the end of the direct pulse wave. The *e* point marks the end of the systolic period (aortic valve closure), and for the purposes of this disclosure it is assumed that the c point marks the start of the reflected wave and the d point marks the end of the reflected wave. The amplitudes of the waveform at these points and the timing of these points relative to the start of a beat interval serve as features characterising the morphology of the PPG beat interval. Changes in the features correlate with BP changes.

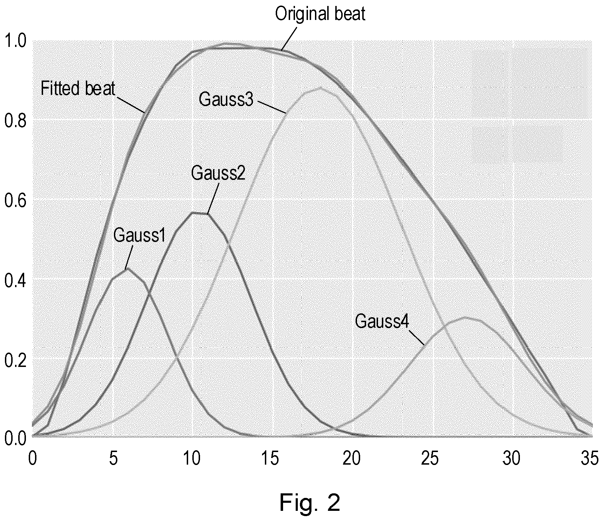

In a second set of methods, pulse decomposition is used to estimate BP. In "Multi-Gaussian fitting for the assessment of left ventricular ejection time from the Photoplethysmogram" by Couceiro et al, 2012 a technique is explained where a PPG beat interval is modelled as a combination of multiple Gaussian-shaped waves. The concept is illustrated in Fig. 2, where a single PPG beat interval is decomposed into four Gaussian shaped functions. The mean and standard deviation of the fitted Gaussians serve as features that parametrise the PPG beat interval. From the fitted Gaussian functions, other features can be extracted that are predictive for the left ventricular ejection time, augmentation index and reflection index. Again, variation in feature values correlate with variations in BP.

In the third set of methods, frequency and irregularity analysis is used to estimate BP. In "Non-invasive estimate of blood glucose and blood pressure from a photoplethysmograph by means of machine learning techniques" by Monte-Moreno, Artificial Intelligence in Medicine, 2011, a method is presented where specific signal processing techniques are applied to the PPG signal to quantify entropy, irregularity of the waveform. The underlying algorithms are Shannon's entropy, Kaiser-teager energy, Qi-Zheng energy and auto-regressive analysis. This set of methods does not extract features for individual inter beat intervals, whereas the first and second set of methods do.

SUMMARY OF THE INVENTION

It is known that respiration activity modulates the PPG waveform. The paper "Developing an algorithm for pulse oximetry derived respiratory rate (RRoxi): a healthy volunteer study" by Addison et al., 2012, explains that respiratory activity can cause the PPG waveform to modulate in three fundamental ways: 1) baseline (DC) modulation - as changes in venous return secondary to changes in intrathoracic pressure throughout the respiratory cycle cause a baseline DC modulation of the PPG signal; 2) pulse amplitude modulation - as decreased left ventricular stroke volume, due to changes in intrathoracic pressure during inspiration, leads to decreased pulse amplitude during this phase of respiration; and 3) respiratory sinus arrhythmia (RSA) - this is a variation in heart rate that occurs throughout the respiratory cycle.

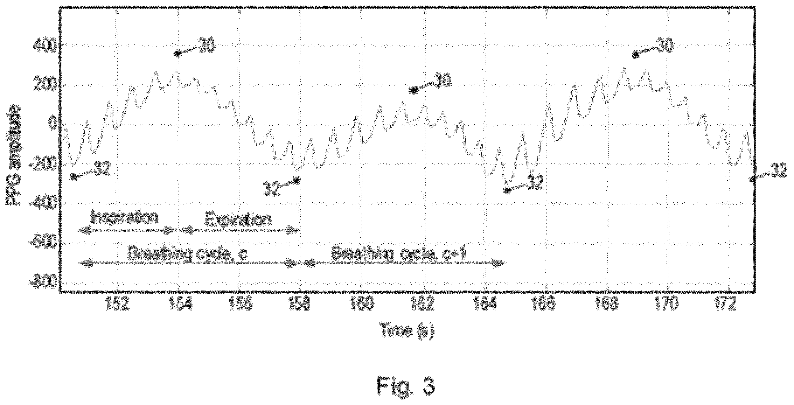

Experimental data also indicates that the morphology of the dicrotic notch (which marks the end of the systolic phase of the cardiac cycle) changes over time in a cyclic pattern due to respiratory effects. The graph in Fig. 3 shows an exemplary PPG signal where respiration activity modulates the morphology of the PPG wave. The points marked 30 in Fig. 3 represent the start of the expiration phase (i.e. breathing out) and the points marked 32 in Fig. 3 represent the start of the inspiration phase (i.e. breathing in). DC modulation, pulse amplitude modulation and deformation of the dicrotic notch corresponding to three breathing cycles are clearly visible in Fig 3. In this example, the PPG sensor was positioned at the wrist.

In US 2019/298195, a non-invasive and comfortable cuffless method is described where a blood pressure dip is predicted with a wearable sensor containing a PPG sensor and an acceleration sensor. The method is an epoch-based approach, meaning that a 24-hour PPG signal is segmented into epochs with a length of one minute. For each epoch, a continuous blood pressure estimate is made based on a combination of PPG morphology features and heart rate variability (HRV) features that are extracted in each epoch. From all continuous blood pressure estimates made within 24 hours, a blood pressure dip is computed by linking epochs belonging to the daytime and nocturnal sleep periods and comparing the average daytime BP level with respect to the average BP during sleep. However, this method does not take the dynamics of respiratory activity within the epoch into account in the process of extracting the PPG morphology features that are eventually used in the prediction of a continuous blood pressure for that epoch. As a result, the BP estimated from the extracted PPG morphology features may be influenced by the respiratory activity, reducing the reliability of the estimated BP.

Therefore, there is a need for improvements in the processing of blood volume signals (e.g. PPG signals) to estimate blood pressure.

Embodiments of the techniques described herein take account of respiratory effort in the process of BP estimation based on inter-beat-interval PPG waveform morphology features. In the feature extraction process, morphology features can be exclusively extracted from beat intervals that occur at a specific (predefined) respiratory phase. This reduces the variation in morphology feature values used for estimating a blood pressure value, as features are only extracted from a specific (and the same) respiration phase. Consequently, the proposed techniques will result into a more robust continuous BP prediction.

According to a first specific aspect, there is provided a computer-implemented method of estimating blood pressure of a subject. The method comprises (i) processing a first blood volume signal to identify a plurality of blood volume signal segments each corresponding to a respective cardiac cycle of the subject, wherein the first blood volume signal comprises measurements of blood volume or changes in blood volume in a body part of the subject in a first time period; (ii) processing a first respiration signal to identify a plurality of respiration cycles of the subject in the first time period, wherein the first respiration signal represents respiratory activity of the subject during the first time period, and wherein each respiration cycle comprises a plurality of respiration cycle phases; (iii) selecting a subset of the plurality of blood volume signal segments, wherein the blood volume signal segments selected for the subset are blood volume signal segments corresponding in time to a same respiration cycle phase in the plurality of respiration cycles; and (iv) estimating blood pressure of the subject based on morphology features of the blood volume signal segments in the subset. Thus, the method according to the first aspect removes the variation caused by respiratory effort in estimating BP based on inter-beat-interval blood volume waveform morphology features, and provides a more robust continuous BP prediction.

In some embodiments, the same respiration cycle phase is one of: a transition from inhaling to exhaling, and a transition from exhaling to inhaling. The use of these respiration cycle phases is useful as they can be easier or more straight forward to detect in the first respiration signal. In these embodiments, the blood volume signal segments selected for the subset can be blood volume signal segments corresponding in time to said one of the transition from inhaling to exhaling and the transition from exhaling to inhaling in respective identified respiration cycles.

In some embodiments, the method further comprises receiving the first blood volume signal from a blood volume sensor.

In some embodiments, the method further comprises receiving the first respiration signal from a respiration sensor. In alternative embodiments, the method further comprises processing the first blood volume signal to determine the first respiration signal. These alternative embodiments have the benefit that a separate respiration sensor is not required.

In some embodiments, the first blood volume signal is a PPG signal.

In some embodiments, the step of processing the first blood volume signal comprises determining a quality measure for parts of the first blood volume signal; and processing parts of the first blood volume signal for which the determined quality measure meets a quality criterion to identify the plurality of blood volume signal segments. In this way, the reliability of the resulting blood pressure estimation can be further improved by excluding lower quality parts of the first blood volume signal. In these embodiments, the quality measure can relate to motion artefacts in the first blood volume signal due to relative movement of the subject and a blood volume sensor that obtains the first blood volume signal. In addition or alternatively, the quality measure can relate to an ambient light level in the first blood volume signal.

In some embodiments, the method further comprises repeating steps (i) to (iv) for a second blood volume signal and a second respiration signal, wherein the second blood volume signal and the second respiration signal relate to the subject in a second time period; comparing the estimated blood pressure for the first time period to the estimated blood pressure for the second time period to estimate a change in blood pressure from the first time period to the second time period. These embodiments have the advantage that blood pressure can be continuously or intermittently measured, and changes in blood pressure observed.

In some embodiments, the morphology features comprise any of: morphology features determined from a first or second derivative with respect to time of the blood volume signal segments in the subset; and a mean and/or standard deviation of Gaussian shaped functions obtained by decomposing the blood volume signal segments in the subset.

According to a second aspect, there is provided a computer program product comprising a computer readable medium having computer readable code embodied therein, the computer readable code being configured such that, on execution by a suitable computer or processing unit, the computer or processing unit is caused to perform the method according to the first aspect or any embodiment thereof.

According to a third aspect, there is provided an apparatus for estimating blood pressure of a subject. The apparatus comprises a processing unit configured to (i) process a first blood volume signal to identify a plurality of blood volume signal segments each corresponding to a respective cardiac cycle of the subject, wherein the first blood volume signal comprises measurements of blood volume or changes in blood volume in a body part of the subject in a first time period; (ii) process a first respiration signal to identify a plurality of respiration cycles of the subject in the first time period, wherein the first respiration signal represents respiratory activity of the subject during the first time period, and wherein each respiration cycle comprises a plurality of respiration cycle phases; (iii) select a subset of the plurality of blood volume signal segments, wherein the blood volume signal segments selected for the subset are blood volume signal segments corresponding in time to a same respiration cycle phase in the plurality of respiration cycles; and (iv) estimate blood pressure of the subject based on morphology features of the blood volume signal segments in the subset. Thus, the apparatus according to the third aspect removes the variation caused by respiratory effort in estimating BP based on inter-beat-interval blood volume waveform morphology features, and provides a more robust continuous BP prediction.

In some embodiments, the same respiration cycle phase is one of: a transition from inhaling to exhaling, and a transition from exhaling to inhaling. The use of these respiration cycle phases is useful as they can be easier or more straight forward to detect in the first respiration signal. In these embodiments, the blood volume signal segments selected for the subset can be blood volume signal segments corresponding in time to said one of the transition from inhaling to exhaling and the transition from exhaling to inhaling in respective identified respiration cycles.

In some embodiments, the processing unit is further configured to receive the first blood volume signal from a blood volume sensor. In some embodiments, the apparatus further comprises the blood volume sensor for obtaining the first blood volume signal. In alternative embodiments, the processing unit is configured to receive the first blood volume signal from a blood volume sensor separate from the apparatus.

In some embodiments, the processing unit is further configured to receive the first respiration signal from a respiration sensor. In some embodiments, the apparatus further comprises the respiration sensor for obtaining the first respiration signal. In alternative embodiments, the processing unit is configured to receive the first respiration signal from a respiration sensor separate from the apparatus.

In alternative embodiments, the processing unit is further configured to process the first blood volume signal to determine the first respiration signal. These alternative embodiments have the benefit that a separate respiration sensor is not required.

In some embodiments, the first blood volume signal is a PPG signal.

In some embodiments, the processing unit is configured to process the first blood volume signal by determining a quality measure for parts of the first blood volume signal; and processing parts of the first blood volume signal for which the determined quality measure meets a quality criterion to identify the plurality of blood volume signal segments. In this way, the reliability of the resulting blood pressure estimation can be further improved by excluding lower quality parts of the first blood volume signal. In these embodiments, the quality measure can relate to motion artefacts in the first blood volume signal due to relative movement of the subject and a blood volume sensor that obtains the first blood volume signal. In addition or alternatively, the quality measure can relate to an ambient light level in the first blood volume signal.

In some embodiments, the processing unit is further configured to: repeat operations (i) to (iv) for a second blood volume signal and a second respiration signal, wherein the second blood volume signal and the second respiration signal relate to the subject in a second time period; compare the estimated blood pressure for the first time period to the estimated blood pressure for the second time period to estimate a change in blood pressure from the first time period to the second time period. These embodiments have the advantage that blood pressure can be continuously or intermittently measured, and changes in blood pressure observed.

In some embodiments, the morphology features comprise any of: morphology features determined from a first or second derivative with respect to time of the blood volume signal segments in the subset; and a mean and/or standard deviation of Gaussian shaped functions obtained by decomposing the blood volume signal segments in the subset. These and other aspects will be apparent from and elucidated with reference to the embodiment(s) described hereinafter.

BRIEF DESCRIPTION OF THE DRAWINGS

Exemplary embodiments will now be described, by way of example only, with reference to the following drawings, in which:

Fig. 1(a) shows a 1-second segment of a PPG signal, and Figs. 1(b) and 1(c) show the first and second derivatives of the PPG signal respectively;
Fig. 2 is a graph illustrating an exemplary Gaussian decomposition of a PPG waveform;
Fig. 3 shows part of a PPG signal and illustrates breathing-induced PPG wave morphology changes;
Fig. 4 illustrates respiratory cycles measured using an airflow sensor in or close to the nasal passages of the subject;
Fig. 5 is a block diagram of an apparatus that can be used to implement the techniques described herein;
Fig. 6 is a functional block diagram illustrating the processing of a blood volume signal to estimate blood pressure according to an exemplary embodiment; and
Fig. 7 is a flow chart illustrating a method of estimating blood pressure according to various embodiments.

DETAILED DESCRIPTION OF EMBODIMENTS

As noted above, the techniques described herein take account of respiratory effort in the process of blood pressure (BP) estimation based on inter-beat-interval PPG waveform morphology features. If a blood volume signal (e.g. a PPG signal) is obtained continuously for a subject, a continuous estimate of BP for the subject can be obtained.

Thus, a sensor system can be used to obtain a blood volume signal. The sensor system can include or be a PPG sensor, and in some embodiments the sensor system can include one or more sensors for measuring respiration activity of the subject. For example, an accelerometer or other type of respiration activity sensor can be provided (e.g. a strain gauge or airflow sensor). The blood volume sensor can be positioned on the body of the subject, for example at the wrist or on a fingertip, or the blood volume sensor can measure the blood volume remotely (e.g. a so-called remote PPG or rPPG sensor). A respiration sensor can be positioned at the chest, although other locations on the body are possible.

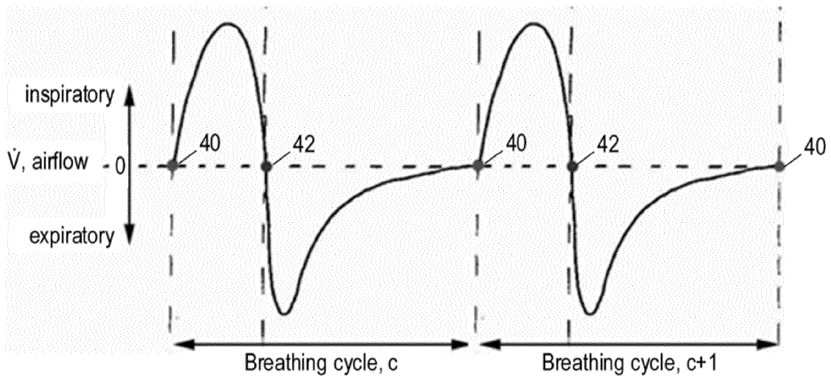

The blood volume signal (and other sensor signals, if present) are input to a processing unit. The processing unit detects the respiratory cycle phases or stages in each respiratory cycle of the subject. Within a respiration cycle the following breathing phases or moments can detected: inhalation and exhalation change points/transitions. Fig. 4 illustrates respiratory cycles measured using an airflow sensor in or close to the nasal passages of the subject, and shows the inhalation start/exhalation end (marked 40) and exhalation start/inhalation end (marked 42) at the times when the airflow is 0.

The processing unit detects the inter-beat-intervals (IBI) in the blood volume signal and computes morphology features within the beat intervals. An inter-beat-interval corresponds to a single cardiac cycle (i.e. heart beat) of the subject. Morphology features for cardiac cycles occurring at the same part of the respiration cycle are used to estimate the blood pressure of the subject. That is, only morphology features of cardiac cycles occurring at an exhalation start phase 42 may be used to estimate BP. In a similar way, it can be that only morphology features of cardiac cycles occurring at an inhalation start phase 40 may be used to estimate BP.

Blood pressure estimation can be performed continuously by combining the extracted respiratory information and morphology features and mapping them to blood pressure estimate, for example using a pre-trained model.

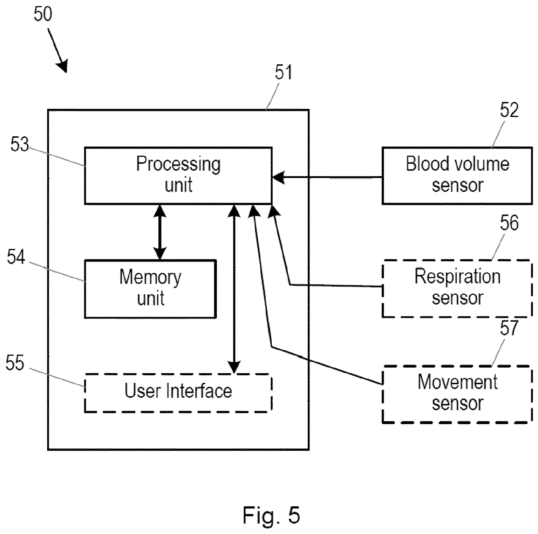

Fig. 5 is a block diagram of a system 50 according to various embodiments for estimating blood pressure of a subject. The system 50 comprises an apparatus 51 that operates according to the techniques described herein to estimate blood pressure from blood volume signals obtained using a blood volume sensor 52.

The blood volume sensor 52 is to measure blood volume in a part of the body of the subject and output a blood volume signal related to the volume of blood passing through that part of the body. The blood volume signal can be a time series of measurement samples representing blood volume over time. Various different types of blood volume sensors can be used to measure blood volume. In some embodiments, the blood volume sensor 52 can be a PPG sensor 52. A PPG sensor 52 can comprise one or more light sources and one or more light sensors. In some embodiments, a remote PPG sensor 52 can be used that comprises a light sensor in the form of a camera or radar sensor that observes the subject or part of the body of the subject from a distance. In other embodiments, the blood volume sensor 52 can be a cuff volume sensor or cuff pressure sensor associated with a cuff that is to be placed around a body part, a strain gauge, a light sensor associated with an optical fibre. The blood volume sensor 52 may be a sensor that is positioned on a part of the body of the subject, such as on the chest, or on a peripheral body location, such as a wrist, fingertip, toe or earlobe. Alternatively, the blood volume sensor 52 can be a sensor that can measure blood volume remotely from the subject, such as embodiments where the blood volume sensor includes a camera.

As known to those skilled in the art, a PPG sensor 52 comprises a light sensor (e.g. a photodiode), and typically one or more light sources (e.g. light emitting diodes (LEDs)). The light sensor can be positioned with respect to the light source(s) so that the light sensor measures the light passing through the body part from the one or more light sources (a so-called transmissive arrangement), or the light sensor can be positioned with respect to the light source(s) so that the light sensor measures the light from the light source(s) that is reflected from the body part (a so-called reflective arrangement). In the case of a chest-worn PPG sensor 52, the PPG sensor 52 may use a reflective arrangement, whereas for a peripheral body location, such as a wrist, fingertip, toe or earlobe, a transmissive arrangement can be used. Alternatively, as noted above, the blood volume sensor 52 can be a remote PPG sensor, such as a camera.

Although the blood volume sensor 52 is shown in Fig. 5 as being separate from the apparatus 51, in alternative embodiments the blood volume sensor 52 may be part of the apparatus 51.

The apparatus 51 may be in the form of, or be part of, a computing device, such as a server, desktop computer, laptop, tablet computer, smartphone, smartwatch, sensor patch, etc. The apparatus 51 includes a processing unit 53 that controls the operation of the apparatus 51 and that can be configured to execute or perform the methods described herein. In particular the processing unit 53 receives the blood volume signal from the blood volume sensor 52.

The processing unit 53 can be implemented in numerous ways, with software and/or hardware, to perform the various functions described herein. The processing unit 53 may comprise one or more microprocessors or digital signal processors (DSPs) that may be programmed using software or computer program code to perform the required functions and/or to control components of the processing unit 53 to effect the required functions. The processing unit 53 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), field-programmable gate arrays (FPGAs), hardware for implementing a neural network and/or so-called artificial intelligence (AI) hardware accelerators (i.e. a processor(s) or other hardware specifically designed for AI applications that can be used alongside a main processor).

The processing unit 53 is connected to a memory unit 54 that can store data, information and/or signals for use by the processing unit 53 in controlling the operation of the apparatus 51 and/or in executing or performing the methods described herein. In some implementations the memory unit 54 stores computer-readable code that can be executed by the processing unit 53 so that the processing unit 53 performs one or more functions, including the methods described herein. In particular embodiments, the program code can be in the form of an application for a smartwatch, smartphone, tablet, laptop or computer. The memory unit 54 can comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM) and electrically erasable PROM (EEPROM), and the memory unit 54 can be implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

In some embodiments, the apparatus 51 comprises a user interface 55 that includes one or more components that enables a user of apparatus 51 to input information, data and/or commands into the apparatus 51, and/or enables the apparatus 51 to output information or data to the user of the apparatus 51. Information that can be output by the user interface 55 can include an estimate of blood pressure and/or an estimate of changes of blood pressure. The user interface 55 can comprise any suitable input component(s), including but not limited to a keyboard, keypad, one or more buttons, switches or dials, a mouse, a track pad, a touchscreen, a stylus, a camera, a microphone, etc., and/or the user interface 55 can comprise any suitable output component(s), including but not limited to a display screen, one or more lights or light elements, one or more loudspeakers, a vibrating element, etc.

As noted above, information about the respiration cycles (and in particular the phases of the respiration cycle) is required in order to implement the methods described herein. In some embodiments, the respiration cycle information is extracted from the blood volume signal. For example, the respiration rate is typically much lower than the pulse rate, and the blood volume signal can be low-pass filtered (with a cut-off frequency below the pulse rate), and the low pass filtered blood volume signal analysed to identify the respiration rate. However, in alternative embodiments, the respiration cycle information is obtained using a separate respiration sensor 56. The respiration sensor 56 can be any suitable type of sensor for measuring respiration (breathing). For example the respiration sensor 56 be an air flow sensor that is positioned in or near the nose or mouth of the subject, an accelerometer that can be worn on or near the chest of the subject to measure movement of the chest during breathing, a strain gauge that is worn as part of a band that is placed around the upper body of the subject, a camera that can observe the subject or a part of the body of the subject (e.g. the chest), or a respiratory inductance PPG (RIP) sensor, etc. A RIP sensor evaluates pulmonary ventilation by measuring the movement of the chest and abdominal wall. Similar to the blood volume sensor 52, the respiration sensor 56 may be separate from the apparatus 51, or part of the apparatus 51.

In some embodiments, the presence of motion artefacts in the blood volume signal can be detected by analysing a signal from a sensor that measures the movement of the subject. This can enable parts of the blood volume signal to be discarded or rejected where the motion is too high or motion artefacts are present. Thus, the system 50 can comprise a movement sensor 57 for measuring the movement of the subject. The movement sensor 57 can be any suitable type of sensor for measuring movement. For example the movement sensor 57 can be an accelerometer, a gyroscope, etc. Similar to the blood volume sensor 52, the movement sensor 57 may be separate from the apparatus 51, or part of the apparatus 51.

In embodiments where the respiration sensor 56 is an accelerometer, the accelerometer can also perform the functions of the movement sensor 57. In some embodiments, respiration cycle information can be obtained from the blood volume signal as described above in conjunction with an acceleration signal from movement sensor 57.

It will be appreciated that a practical implementation of an apparatus 51 may include additional components to those shown in Fig. 5. For example the apparatus 51 may also include a power supply, such as a battery, or components for enabling the apparatus 51 to be connected to a mains power supply. The apparatus 51 may also include interface circuitry for enabling a data connection to and/or data exchange with other devices or sensors. For example, in embodiments where the blood volume sensor 52 is separate from the apparatus 51, the blood volume signal can be received from the blood volume sensor 52 via the interface circuitry. Furthermore, in some embodiments the system 50 can be for measuring additional physiological characteristics of the subject, in which case the system 50 can comprise one or more additional sensors that are used to measure these physiological characteristics of the subject.

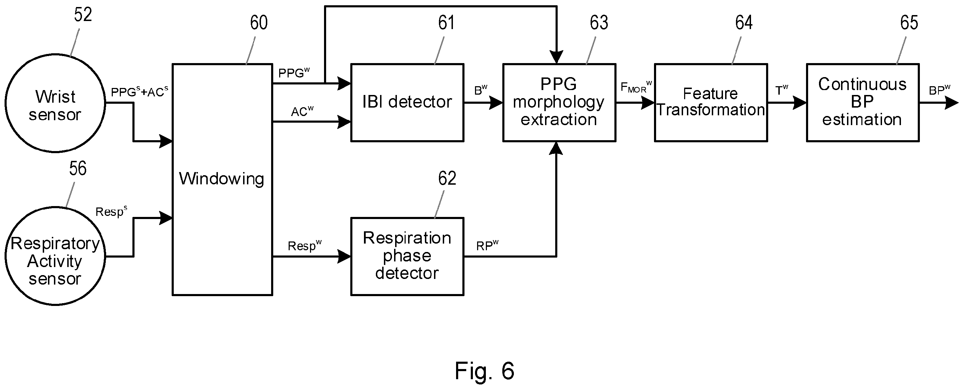

A specific exemplary embodiment of the techniques presented herein is described below with reference to the functional block diagram shown in Fig. 6. Each block in Fig. 6 represents a particular stage of the processing of a blood volume signal to estimate blood pressure. It will be appreciated that in practice one or more processing units can implement the functions represented by the functional blocks. In this exemplary embodiment, the blood volume sensor 52 is a PPG sensor 52. The PPG sensor 52 is part of a sensor unit worn on the wrist of the subject, and the sensor unit also includes an accelerometer. A separate respiration sensor 56 in the form of a RIP sensor 56 is also provided. The PPG sensor 52 applies the concept of pulse oximetry which tracks the average skin tone, as skin tone varies with blood volume and blood oxygenation. The PPG sensor 52 shines light (e.g. using an LED) onto the skin surface and measures the amount of light reflected by the skin, for example using a photodiode. During each cardiac cycle the amount of reflected light varies as more or less blood flows through the arterioles. The variation in skin tone over time contains information on the blood volume and the light sensor converts the reflected light into an electrical signal. The blood volume signal output by the PPG sensor 52 and the respiration signal output by the respiration sensor 56 are provided to the processing unit 53 for subsequent processing to estimate blood pressure. The blood volume signal and the respiration signal relate to the same time period. It will be appreciated that although the functional block diagram in Fig. 6 relates to embodiments that include a PPG sensor 52 and RIP sensor 56, the processing described below can be applied to other types of blood volume sensor 52 and/or other types of respiration sensor 56.

Sections of the blood volume signal (denoted $PPG^s$), acceleration signal (denoted $AC^s$) and respiration signal (denoted $Resp^s$) for a particular epoch or time frame are processed to estimate blood pressure. Thus the signals are input to a windowing block 60. In the windowing block 60, cotemporaneous sections of the raw acceleration signal $AC^s$, raw PPG signal $PPG^s$ and respiratory activity signal $Resp^s$ with a predetermined length, for example one minute, are selected and output to the next functional block. The $w^{th}$ time frame (window) in $AC^s$, $PPG^s$ and $Resp^s$ are denoted by $AC^w$, $PPG^w$, and $Resp^w$, respectively. The subsequent functional blocks operate on the signal for the $w^{th}$ time frame. After outputting the signals for the $w^{th}$ time frame, the windowing block 60 selects and outputs the signals for the $(w+1)^{th}$ time frame. For continuous monitoring of blood pressure, the signals in the $(w+1)^{th}$ time frame are preferably non-overlapping but contiguous with the signals in the $w^{th}$ time frame. Alternatively the signals in the $(w+1)^{th}$ time frame may be non-contiguous with the signals in the $w^{th}$ time frame. In another alternative, the signals in the $(w+1)^{th}$ time frame may partially overlap with the signals in the $w^{th}$ time frame.

The $AC^w$ and $PPG^w$ are input to an inter-beat-interval (IBI) detection block 61, and the $Resp^w$ is input to a respiration phase detection block 62.

The IBI detection block 61 detects the cardiac cycles (IBIs) in the $PPG^w$ signal. The IBI detection block 61 can detect cardiac cycles by detecting the heart beats in the $PPG^w$ signal. A cardiac cycle is considered to be the interval between two successive heart beat locations in the $PPG^w$ signal. Thus the onset of a cardiac cycle is considered to be a heart beat and cardiac cycles can be found by identifying the individual beat locations in the $PPG^w$ signal. Those skilled in the art will be aware of various methods of identifying beat locations in a PPG signal. One such method for beat localisation is known as the 'intersecting tangents' method, as described in the paper "Comparison of foot finding methods for deriving instantaneous pulse rates from photoplethysmographic signals" by Hemon, M. C. & Phillips, J. P. (2015), Journal of Clinical Monitoring and Computing, 30(2), pp. 157-168. In this method, for a $PPG^w$ signal, the onset of a cardiac cycle (heart beat) is defined as the point yielded by the intersection of a line tangent to the maximum of the initial upslope (i.e. maximum of the 1$^{st}$ derivative) and a line tangent to the preceding minimum value.

Each part of the PPG signal corresponding to a detected cardiac cycle is referred to herein as a PPG signal segment (or more generally as a blood volume signal segment).

In some embodiments, the IBI detection block 61 can evaluate the quality of any detected cardiac cycles, and reject low quality beats/cardiac cycles. For example, the PPG signal segments can be evaluated for the presence or influence of ambient light (for example as indicated by a DC component in the PPG signal segment), and any PPG signal segments rejected that have ambient light higher than a threshold value. In addition, or alternatively, the amount of motion of the subject at the time that the relevant part of the PPG signal was measured can be evaluated to determine if motion artefacts may be influencing the measured PPG signal. The motion of the subject can be determined from the acceleration signal, $AC^w$. In a simple example, if the magnitude of the acceleration is above a threshold for a particular PPG signal segment, the presence of motion artefacts can be assumed, and that PPG signal segment rejected.

A set of beats/cardiac cycles remaining after rejecting any low-quality beats/cardiac cycles are output by the IBI detection block 61 to a PPG morphology extraction block 63. Thus, for the high quality beats detected within the $w^{th}$ epoch (time period), IBI's are constructed defined by the set:

$$\boldsymbol{B}^w = [b^{w,1}, b^{w,m}, \ldots, b^{w,M}], \tag{1}$$

and are output by the IBI detection block 61 to the PPG morphology extraction block 63. The number of elements in set $B^w$, denoted by M, varies per epoch, as the number of inter beat intervals detected within an epoch varies, and the number of cardiac cycles rejected for being of low quality also varies.

As noted above, the windowed respiration activity signal $Resp^w$ is output by the windowing block 60 to respiration phase detection block 62. The respiration phase detection block 62 analyses the windowed respiration activity signal $Resp^w$ to identify respiration cycles of the subject, and in particular respiration cycle phases, such as transition points from inhaling to exhaling, or transition points from exhaling to inhaling.

Firstly, the respiration phase detection block 62 segments the respiration signal $Resp^w$ into individual respiration cycles r to give the set:

$$\boldsymbol{R}^w = [r^{w,1}, r^{w,c}, \ldots, r^{w,C}], \tag{2}$$

where C refers to the number of respiration cycles detected within the $w^{th}$ epoch. It should be noted that the number of elements in set $R^w$ can vary per analysed epoch as the number of respiration cycles occurring within an epoch varies due to the subject's varying breathing pace.

Next, for each $c^{th}$ respiration cycle $r^c$ in the $w^{th}$ epoch, the respiration phase detection block 62 detects the start of the inspiration $i_s^{w,c}$, which is defined as

$$i_s^{w,c} = \text{DetectInspiration}(r^{w,c}). \tag{3}$$

The respiration phase detection block 62 outputs a data structure $RP^w$ that comprises the set of time instances that mark the start of inspiration phases detected within the $w^{th}$ epoch, defined by

$$\boldsymbol{RP^w} = [i_s^{w,1}, i_s^{w,c}, \ldots, i_s^{w,C}]. \tag{4}$$

It will be appreciated that in alternative embodiments the respiration phase detection block 62 can detects a different phase of the respiration cycle, such as the start of the exhalation phase.

The data structure $RP^w$ is output by the respiration phase detection block 62 to the PPG morphology extraction block 63.

Thus the PPG morphology extraction block 63 receives the beat signal $B^w$, the windowed PPG signal, $PPG^w$, and the data structure $RP^w$ indicating the timing of the start of each inspiration phase in the windowed respiration signal. The PPG morphology extraction block 63 operates on the received signals and information to select a subset of the cardiac cycles indicated in the beat signal $B^w$ that correspond in time to the start of each inspiration phase indicated in $RP^w$. Thus, for the start of each inspiration phase, a respective cardiac cycle is selected from $B^w$. The selected cardiac cycle can be the cardiac cycle during which the inspiration phase starts, the last full cardiac cycle immediately before the inspiration phase starts, or the first full cardiac cycle immediately after the inspiration phase starts. The PPG morphology extraction block 63 then estimates the blood pressure of the subject based on morphology features of the segments of the PPG signal corresponding to the selected subset of cardiac cycles.

The above operations of the PPG morphology extraction block 63 to compute a set of selected cardiac cycles, denoted $D^w$, can be described in algorithmic form as follows:

*Input*: $\boldsymbol{RP^w}$, $\boldsymbol{B^w}$

*Output*: $\boldsymbol{D^w}$

$\boldsymbol{D^w} = \{\}$

*For all* $i_s^{w,c} \in \boldsymbol{RP^w}$,

$\quad d^{w,c} = SelectLastIBIExh(B^w, i_s^{w,c}), \text{ and } d^{w,c} \in \boldsymbol{B^w}$

$\boldsymbol{D^w} = append(\boldsymbol{D^w}, d^{w,c})$

Here, the function *SelectLastIBIExh* returns the last detected beat interval of the previous respiration cycle that is sufficiently close to $i_s^{w,c}$ according to some threshold $T_h$. Typically, $d^{w,c}$ refers to the inter beat interval (cardiac cycle) marking the end of the exhalation phase of the previous respiration cycle.

Having selected cardiac cycles that occur at the same part of the respiration cycle, the PPG morphology extraction block 63 computes the feature set $F_{MOR}^w$ with the function:

$$F_{MOR}^w = ExtractMorphFeat(\boldsymbol{D^w}, PPG^w) \tag{5}$$

In short, function *ExtractMorphFeat* extracts features that characterise the PPG wave morphology of the inter beat intervals (cardiac cycles) that correspond to the end of the expiration phase.

In particular embodiments, feature set $F_{MOR}^w$ can be computed as follows. For each inter beat interval $d^{w,c}$, a number K of morphology features $f^{w,k,c}$ are computed where $k = 1 \ldots K$. The morphology feature set computed for each interval $d^{w,c}$ can comprise landmark features and/or pulse decomposition features of the PPG signal corresponding to the cardiac cycle as described in the Background section. For example, the amplitudes and/or timing of one or more of the reference points *a, b, c, d* and *e* shown in Fig. 1 can be determined for each PPG signal segment corresponding to a selected cardiac cycle. In addition or alternatively, each PPG signal segment can be modelled as multiple Gaussian-shaped waves, and the mean and standard deviation of the fitted Gaussians parameterise the PPG signal segment.

Next, for each $k^{th}$ feature, the PPG morphology extraction block 63 aggregates the computed feature values across all $C$ respiration cycles within epoch $w$. Subsequently, the PPG morphology extraction block 63 computes statistical features from the aggregated features such as, for example, mean, median and standard deviation, to give the set:

$$F^{w,k} = stats([f^{w,k,1}, f^{w,k,c}, ..., f^{w,k,C}]). \qquad (6)$$

Doing this for all K features yields the feature set

$$F_{MOR}^{w} = [F^{w,1}, F^{w,k}, ..., F^{w,K}]. \qquad (7)$$

Feature output by the PPG morphology extraction block 63 to a feature transformation block 64 that applies feature normalisation to reduce the within-subject and between-subject variation. Thus the feature transformation block 64 aims to normalise features to eliminate outliers and standardise the feature value ranges. Types of normalisation that can be applied include, for instance, amplitude normalisation, percentile normalisation, z-score standardisation, Winsorisation, power transforms, etc. The set $T^w$ represents the transformed feature set that is output by the feature transformation block 64 to a blood pressure estimation block 65.

The blood pressure estimation block 65 determines a blood pressure estimate $BP^w$ for the $w^{th}$ epoch based on the transformed features $T^w$ and a pre-trained blood pressure estimation model $P_{bp}$ according to:

$$BP^w = P_{bp}(T^w). \qquad (8)$$

The aim of the blood pressure estimation model $P_{bp}$ is to map the (transformed) input feature set, $F_{MOR}^{w}$, to a blood pressure estimate using machine learning technologies. The model can be trained using an offline data driven training stage in which a cost function is used to minimise the error between a model-estimated BP value and a reference BP value. The model can be, for example, a dense neural net, a deep neural net, a long- and short-term memory (LSTM) neural network, a random forest or a linear regression model. In order to train a robust BP estimation model (in terms of generalisation error), a sufficient amount of training data is required, i.e. large quantities of PPG measurement data and reference BP values obtained from many different subjects.

In some embodiments, additional demographic information can be included in the prediction model, which would give:

$$BP^w = P_{bp}(T^w, SubInf), \qquad (9)$$

where *SubInf* represents demographic information of the relevant subject such as age, body mass index (BMI), height, weight, gender, etc. The model $P_{bp}$ that maps the transformed features to a blood pressure value can be trained in an offline training stage.

Alternatively, the blood pressure estimation block 65 can implement a parameterised model or mathematical formula to estimate blood pressure from the transformed features or transformed feature values. A calibration procedure for the parameterised model or mathematical formula may be performed based on known blood pressure values.

The techniques described herein can be applied in a number of different monitoring scenarios. For example, the blood pressure estimation technique can be used in outpatient monitoring, remote patient monitoring, vital signs monitoring, continuous blood pressure estimation and blood pressure dip estimation (i.e. estimating if the blood pressure is going to or is suddenly dropping to dangerous levels).

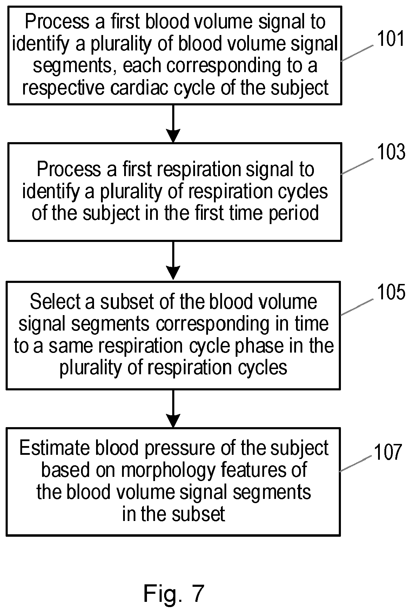

The flow chart in Fig. 7 illustrates a method for estimating blood pressure for a subject according to various embodiments. The method can be performed by the apparatus 51, for example by the processing unit 53. The processing unit 53 may perform the method as a result of executing suitably configured computer readable code.

Initially, the processing unit 53 receives or obtains a first blood volume signal for a subject. The first blood volume signal comprises measurements of blood volume or changes in blood volume in a body part of the subject in a first time period. The first time period may be of the order of 1 minute, although the first time period may be shorter or much longer than this. The body part that the blood volume signal relates to may be the chest, forehead, or a peripheral body part such as the wrist, earlobe, fingertip or toe. The first blood volume signal may have been obtained by a blood volume sensor 52 in contact with the body part, or it may have been obtained by a blood volume sensor 52 remote from the body part (e.g. a camera). In some embodiments, the first blood volume signal is a PPG signal that is obtained by a PPG sensor. In some embodiments, the processing unit 53 can receive or obtain the first blood volume signal directly from blood volume sensor 52 as the blood volume signal is generated. These embodiments enable the blood pressure to be estimated in real time, and optionally continuously. In other embodiments, the first blood volume signal may be stored in the memory unit 54, and the processing unit 53 can retrieve the first blood volume signal from the memory unit 54.

In step 101, the first blood volume signal is processed to identify a plurality of blood volume signal segments each corresponding to a respective cardiac cycle of the subject. To identify a blood volume signal segment, a cardiac cycle or a part of a cardiac cycle is identified in the blood volume signal. As noted above, a cardiac cycle can be considered to be the interval between two successive heart beat locations in the blood volume signal. Thus blood volume signal segments can be identified by identifying individual heart beat locations in the blood volume signal. Also as noted above, those skilled in the art will be aware of various methods of identifying beat locations in a blood volume signal, particularly since conventional heart rate monitors can typically operate based on blood volume signals, such as PPG signals.

In some embodiments of step 101, one or more quality measures can be determined for different parts of the first blood volume signal. One quality measure can relate to the presence of motion artefacts in a particular part of the first blood volume signal due to relative movement of the subject and the blood volume sensor 52 at the time that part of the blood volume signal was obtained. This quality measure can be determined from a signal from a movement sensor 57 that measures the movement of the subject. In the case of a blood volume sensor 52 in the form of a PPG sensor, the or another quality measure can relate to or be ambient light level in part of the first PPG signal. The ambient light level for a particular part of the first PPG signal can be determined as a DC component in that part of the first PPG signal. Any parts of the first blood volume signal for which the determined quality measure(s) do not meet a quality criterion are disregarded when identifying the plurality of blood volume signal segments. Thus, parts of the first blood volume signal for which the determined quality measure(s) meet a quality criterion are processed in step 101 to identify the plurality of blood volume signal segments. For a quality measure relating to motion artefacts, the quality criterion can be met if the motion artefacts are below a threshold level. For a quality measure relating to ambient light, the quality criterion can be met if the ambient light (or DC component) is below a threshold level.

In step 103, a first respiration signal is processed to identify a plurality of respiration cycles of the subject in the first time period. The first respiration signal represents respiratory activity of the subject during the first time period. Each respiration cycle comprises a plurality of respiration cycle phases. For example, each respiratory cycle comprises the start of the inhalation phase (also referred to as the transition from exhalation to inhalation), the inhalation phase, the start of exhalation phase (also referred to as the transition from inhalation to exhalation), and the exhalation phase.

In some embodiments, the first respiration signal is generated by a respiration sensor 56. The respiration sensor 56 can be any of: an air flow sensor, an accelerometer, a strain gauge, a camera, or a RIP sensor. The processing unit 53 can receive or obtain the first respiration signal directly from the respiration sensor 56 as the respiration signal is generated. If the first blood volume signal is also received as it is generated, these embodiments enable the blood pressure to be estimated in real time, and optionally continuously. In other embodiments, the first respiration signal may be stored in the memory unit 54, and the processing unit 53 can retrieve the first respiration signal from the memory unit 54.

In alternative embodiments, the first respiration signal can be determined from the first blood volume signal. In particular, as the respiration rate is typically much lower than the pulse rate (e.g. respiration rate is typically 12 to 16 breaths per minute, whereas pulse rate is typically 60 beats per minute or higher, the blood volume signal can be low-pass filtered with a cut-off frequency below the pulse rate, e.g. the cut-off frequency could be of the order of 0.5 Hz (i.e. around 30 per minute). The low-pass filtered blood volume signal can be analysed to identify the respiration rate.

In step 105, a subset of the plurality of blood volume signal segments are selected. The blood volume signal segments selected for the subset are those blood volume signal segments corresponding in time to a same respiration cycle phase in the plurality of respiration cycles. That is, a respective blood volume signal segment is selected for each of the identified respiration cycles. The blood volume signal segments that are selected all relate to the same respiration cycle phase, such as the start of the inhalation phase (i.e. the transition from exhalation to inhalation), or the start of the exhalation phase (i.e. the transition from inhalation to exhalation).

In an example, the relevant respiration cycle phase is the start of the inhalation phase. Thus, for a first identified respiratory cycle, a first blood volume signal segment is selected that corresponds in time to the start of the inhalation for the first identified respiratory cycle. For the next (second) identified respiratory cycle, a second blood volume signal segment is selected that corresponds in time to the start of the inhalation for the second identified respiratory cycle, and so on. In this way, the blood volume signal segments selected for the subset all took place at the same point in the respiration cycle. In this way, the influence of respiratory activity on the blood volume signal is removed, thereby improving the reliability of the blood pressure estimated in the next step of the method.

Then, in step 107, blood pressure of the subject is estimated based on morphology features of the blood volume signal segments in the subset. That is, the processing unit 53 analyses or processes each blood volume signal segment in the subset to determine respective values of morphology features, and estimates the blood pressure from those values. The morphology features may be determined from a first or second derivative with respect to time of the blood volume signal segments in the subset. Alternatively or in addition, the morphology features may be a mean and/or standard deviation of Gaussian-shaped functions obtained by decomposing the blood volume signal segments in the subset.

In some embodiments, the method further comprises repeating steps 101, 103, 105 and 107 for a second blood volume signal and a second respiration signal that relate to the same subject in a second time period after the first time period. The second time period may be contiguous with the first time period, or the second time period may be much later than the first time period, e.g. 1 hour later or 1 day later. In these embodiments, the processing unit 53 can compare the estimated blood pressure for the first time period to the estimated blood pressure for the second time period to estimate a change in blood pressure for the subject from the first time period to the second time period.

Therefore, there is provided improvements in the processing of blood volume signals to estimate blood pressure, and in particular the influence of respiratory activity on the blood volume signal is removed before estimating the blood pressure.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1.   A computer-implemented method of estimating blood pressure of a subject, the method comprising:

   (i) processing (101) a first blood volume signal to identify a plurality of blood volume signal segments each corresponding to a respective cardiac cycle of the subject, wherein the first blood volume signal comprises measurements of blood volume or changes in blood volume in a body part of the subject in a first time period;
   (ii) processing (103) a first respiration signal to identify a plurality of respiration cycles of the subject in the first time period, wherein the first respiration signal represents respiratory activity of the subject during the first time period, and wherein each respiration cycle comprises a plurality of respiration cycle phases;
   (iii) selecting (105) a subset of the plurality of blood volume signal segments, wherein the blood volume signal segments selected for the subset are blood volume signal segments corresponding in time to a same respiration cycle phase in the plurality of respiration cycles; and
   (iv) estimating (107) blood pressure of the subject based on morphology features of the blood volume signal segments in the subset.

2.   A method as claimed in claim 1, wherein the same respiration cycle phase is one of: a transition from inhaling to exhaling, and a transition from exhaling to inhaling.

3.   A method as claimed in claim 2, wherein the blood volume signal segments selected for the subset are blood volume signal segments corresponding in time to said one of the transition from inhaling to exhaling and the transition from exhaling to inhaling in respective identified respiration cycles.

4.   A method as claimed in any of claims 1-3, wherein the step of processing (103) the first blood volume signal comprises:

   determining a quality measure for parts of the first blood volume signal; and
   processing parts of the first blood volume signal for which the determined quality measure meets a quality criterion to identify the plurality of blood volume signal segments.

5.   A method as claimed in claim 4, wherein the quality measure relates to: motion artefacts in the first blood volume signal due to relative movement of the subject and a blood volume sensor that obtains the first blood volume signal, and/or an ambient light level in the first blood volume signal.

6.   A method as claimed in any of claims 1-5, wherein the method further comprises:

   repeating steps (i) to (iv) for a second blood volume signal and a second respiration signal, wherein the second blood volume signal and the second respiration signal relate to the subject in a second time period; and
   comparing the estimated blood pressure for the first time period to the estimated blood pressure for the second time period to estimate a change in blood pressure from the first time period to the second time period.

7.   A method as claimed in any of claims 1-6, wherein the morphology features comprise any of:

morphology features determined from a first or second derivative with respect to time of the blood volume signal segments in the subset; and

a mean and/or standard deviation of Gaussian shaped functions obtained by decomposing the blood volume signal segments in the subset.

8. A computer program product comprising a computer readable medium having computer readable code embodied therein, the computer readable code being configured such that, on execution by a suitable computer or processing unit, the computer or processing unit is caused to perform the method of any of claims 1-7.

9. An apparatus (51) for estimating blood pressure of a subject, the apparatus (51) comprising a processing unit (53) configured to:

(i) process a first blood volume signal to identify a plurality of blood volume signal segments each corresponding to a respective cardiac cycle of the subject, wherein the first blood volume signal comprises measurements of blood volume or changes in blood volume in a body part of the subject in a first time period;
(ii) process a first respiration signal to identify a plurality of respiration cycles of the subject in the first time period, wherein the first respiration signal represents respiratory activity of the subject during the first time period, and wherein each respiration cycle comprises a plurality of respiration cycle phases;
(iii) select a subset of the plurality of blood volume signal segments, wherein the blood volume signal segments selected for the subset are blood volume signal segments corresponding in time to a same respiration cycle phase in the plurality of respiration cycles; and
(iv) estimate blood pressure of the subject based on morphology features of the blood volume signal segments in the subset.

10. An apparatus (51) as claimed in claim 9, wherein the same respiration cycle phase is one of: a transition from inhaling to exhaling, and a transition from exhaling to inhaling.

11. An apparatus (51) as claimed in claim 10, wherein the blood volume signal segments selected for the subset are blood volume signal segments corresponding in time to said one of the transition from inhaling to exhaling and the transition from exhaling to inhaling in respective identified respiration cycles.

12. An apparatus (51) as claimed in any of claims 9-11, wherein the processing unit (53) is configured to process the first blood volume signal by:

determining a quality measure for parts of the first blood volume signal; and
processing parts of the first blood volume signal for which the determined quality measure meets a quality criterion to identify the plurality of blood volume signal segments.

13. An apparatus (51) as claimed in claim 12, wherein the quality measure relates to: motion artefacts in the first blood volume signal due to relative movement of the subject and a blood volume sensor (52) that obtains the first blood volume signal, and/or an ambient light level in the first blood volume signal.

14. An apparatus (51) as claimed in any of claims 9-13, wherein the processing unit (53) is further configured to:

repeat operations (i) to (iv) for a second blood volume signal and a second respiration signal, wherein the second blood volume signal and the second respiration signal relate to the subject in a second time period; and
compare the estimated blood pressure for the first time period to the estimated blood pressure for the second time period to estimate a change in blood pressure from the first time period to the second time period.

15. An apparatus (51) as claimed in any of claims 9-14, wherein the morphology features comprise any of:

morphology features determined from a first or second derivative with respect to time of the blood volume signal segments in the subset; and
a mean and/or standard deviation of Gaussian shaped functions obtained by decomposing the blood volume signal segments in the subset.

Fig. 1

EP 4 029 437 A1

Fig. 2

EP 4 029 437 A1

Fig. 3

50

51

53

52

Processing
unit

Blood volume
sensor

56

54

Memory
unit

Respiration
sensor

57

55

User Interface

Movement
sensor

Fig. 5

Fig. 6

Process a first blood volume signal to identify a plurality of blood volume signal segments, each corresponding to a respective cardiac cycle of the subject — 101

Process a first respiration signal to identify a plurality of respiration cycles of the subject in the first time period — 103

Select a subset of the blood volume signal segments corresponding in time to a same respiration cycle phase in the plurality of respiration cycles — 105

Estimate blood pressure of the subject based on morphology features of the blood volume signal segments in the subset — 107

Fig. 7

Europäisches
Patentamt

European
Patent Office

Office européen
des brevets

## EUROPEAN SEARCH REPORT

Application Number

EP 21 15 1401

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/199893 A1 (HÜBNER THOMAS [DE]) 19 July 2018 (2018-07-19) * figures 1,2A,3A,3B * * paragraph [0091] - paragraph [0098] * * paragraph [0105] * * paragraph [0110] - paragraph [0112] * * paragraph [0127] - paragraph [0129] * ----- | 1-15 | INV. A61B5/024 A61B5/021 A61B5/08 A61B5/0205 A61B5/00 |
| X | US 2017/196469 A1 (HAN WEI-RU [TW] ET AL) 13 July 2017 (2017-07-13) * figure 6 * * paragraph [0046] - paragraph [0048] * * paragraph [0064] - paragraph [0068] * ----- | 1-3, 6-11,14, 15 | |
| A | US 2005/038349 A1 (CHOI KI-WAN [KR] ET AL) 17 February 2005 (2005-02-17) * paragraph [0083] * ----- | 5,13 | |
| A,D | US 2019/298195 A1 (DE GROOT KOEN THEO JOHAN [NL] ET AL) 3 October 2019 (2019-10-03) * paragraph [0050] - paragraph [0055] * ----- | 5,13 | TECHNICAL FIELDS SEARCHED (IPC) A61B |

The present search report has been drawn up for all claims

| Place of search | Date of completion of the search | Examiner |
|---|---|---|
| The Hague | 25 June 2021 | Oancea, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 15 1401

This annex lists the patent family members relating to the patent documents cited in the above-mentioned European search report.
The members are as contained in the European Patent Office EDP file on
The European Patent Office is in no way liable for these particulars which are merely given for the purpose of information.

25-06-2021

| Patent document cited in search report | Publication date | Patent family member(s) | Publication date |
|---|---|---|---|
| US 2018199893 A1 | 19-07-2018 | AU 2016293110 A1 | 15-02-2018 |
| | | CA 2992508 A1 | 19-01-2017 |
| | | CN 107920763 A | 17-04-2018 |
| | | EP 3117766 A1 | 18-01-2017 |
| | | EP 3117767 A1 | 18-01-2017 |
| | | JP 6798671 B2 | 09-12-2020 |
| | | JP 2018525188 A | 06-09-2018 |
| | | KR 20180029072 A | 19-03-2018 |
| | | RU 2018101306 A | 16-08-2019 |
| | | US 2018199893 A1 | 19-07-2018 |
| | | WO 2017009465 A1 | 19-01-2017 |
| US 2017196469 A1 | 13-07-2017 | TW 201725006 A | 16-07-2017 |
| | | US 2017196469 A1 | 13-07-2017 |
| US 2005038349 A1 | 17-02-2005 | EP 1506736 A2 | 16-02-2005 |
| | | JP 2005058766 A | 10-03-2005 |
| | | KR 20050017254 A | 22-02-2005 |
| | | US 2005038349 A1 | 17-02-2005 |
| US 2019298195 A1 | 03-10-2019 | EP 3773155 A1 | 17-02-2021 |
| | | US 2019298195 A1 | 03-10-2019 |
| | | WO 2019185392 A1 | 03-10-2019 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2019298195 A

**Non-patent literature cited in the description**

- **MONTE-MORENO.** Non-invasive estimate of blood glucose and blood pressure from a photoplethysmograph by means of machine learning techniques. *Artificial Intelligence in Medicine,* 2011
- **ADDISON et al.** *Developing an algorithm for pulse oximetry derived respiratory rate (RRoxi): a healthy volunteer study,* 2012
- **HEMON, M. C. ; PHILLIPS, J. P.** Comparison of foot finding methods for deriving instantaneous pulse rates from photoplethysmographic signals. *Journal of Clinical Monitoring and Computing,* 2015, vol. 30 (2), 157-168